# EUROPEAN PATENT APPLICATION

(11) **EP 0 938 877 A2**
(43) Date of publication of application: **01.09.1999**
(21) Application number: 99301331.7
(22) Date of filing: 24.02.1999
(51) Int. Cl.: A61F 2/06

(54) **Stent crimping device and method of use**

(30) Priority: 25.02.1998 US 30261
(71) Applicant: Advanced Cardiovascular Systems, Inc., Santa Clara, California 95054 (US)
(72) Inventor: Morales, Stephen A., Mountain view, CA 94041 (US)
(74) Representative: McLeish, Nicholas Alistair Maxwell

(57) **Abstract**

A slidably-engageable device for enabling effective crimping of an intravascular stent onto a balloon catheter assembly. The stent crimping device includes at least one compressible and releasable loop portion which enables the stent and catheter assembly to be supported thereon, and is compressible radially inwardly to effectively crimp the stent onto the balloon catheter assembly.

## Description

### BACKGROUND OF THE INVENTION

### Field of the Invention

This invention relates to a stent crimping device of the type that will enable the user to crimp a stent onto the distal end of a balloon catheter assembly, for example of the kind used in a typical percutaneous transluminal coronary angioplasty (PTCA) procedure.

### Description of the Related Art

In a typical PTCA procedure for compressing lesion plaque against the artery wall to dilate the artery lumen, a guiding catheter is introduced percutaneously into the cardiovascular system of a patient through the brachial or femoral arteries and is advanced through the vasculature until the distal end thereof is in the ostium. A guide wire and a dilatation catheter having a balloon on the distal end are introduced through the guiding catheter with the guide wire sliding within the dilatation catheter. The guide wire is first advanced out of the guiding catheter into the patient's coronary vasculature, and the dilatation catheter is advanced over the previously advanced guide wire until the dilatation balloon is positioned properly across the lesion. Once in position across the lesion, a flexible, expandable, pre-formed balloon is inflated to a pre-determined size at relatively high pressures to radially compress the atherosclerotic plaque of the lesion against the inside of the artery wall and to thereby dilate the lumen of the artery. The balloon then is deflated to a small profile, so that the dilatation catheter can be withdrawn from the patient's vasculature and blood flow resumed through the dilated artery. While this procedure is typical, it is not the only method used in angioplasty. Other methods to open a vessel are known, such as atherectomies and plaque-dissolving drugs.

In angioplasty procedures of the kind referenced above, a restenosis of the artery may develop over several months, which may require another angioplasty procedure, a surgical bypass operation, or some method of repairing or strengthening the area. To reduce the chance of the development of restenosis and strengthen the area, a physician can implant an intravascular prosthesis for maintaining vascular patency, typically called a stent. A stent is a device used to hold tissue in place in a vessel or to provide a support for a vessel to hold the vessel open so that blood flows freely. A vanety of devices are known in the art for use as stents, including expandable tubular members, in a variety of patterns, that are able to be crimped onto a balloon catheter and then expanded after being positioned intraluminally on the balloon catheter, and which retain their expanded form. Typically, the stent is loaded and crimped onto the balloon portion of the catheter and advanced to a location inside the artery at the lesion. The stent then is expanded to a larger diameter by the balloon portion of the catheter to implant the stent in the artery at the lesion. Examples of stents and delivery catheters as described are disclosed in more detail in U.S. Patent Nos. 5,102,417 (Palmaz), 5,569,295 (Lam), and 5,514,154 (Lau et al.).

However, if the stent is not effectively crimped onto the catheter balloon portion, when the catheter is advanced in the patient's vasculature the stent may move or even slide off the catheter balloon portion in the body lumen or coronary artery prior to expansion, and may block the flow of blood, requiring procedures to remove the stent.

In procedures where the stent is placed over the balloon portion of the catheter, the stent must be crimped onto the balloon portion to prevent the stent from sliding off the catheter when the catheter is advanced in the patient's vasculature. In the past, this crimping often was done by hand, which does not provide optimum results due to the uneven force being applied, and non-uniform crimps would result. In addition, it was difficult to judge when a uniform and reliable crimp had been applied. The prior art tools and methods have not been entirely adequate in achieving a uniform crimp. Stent designs generally are based on a uniform metal-to-artery ratio for the highest success rate, thus a non-uniformly crimped stent may result in an unevenly expanded stent in the vessel or artery, which is undesirable.

### SUMMARY OF THE INVENTION

This invention is directed to a vascular prosthesis crimping device which enables uniform and tight crimping of a stent onto a catheter balloon portion, to better secure the stent onto the catheter for delivery of the stent through the patient's vasculature. The present invention attempts to solve several problems associated with crimping stents onto balloon catheters.

In an exemplary embodiment ofthe present invention, the stent crimping device includes a compressible and releasable loop portion of a flexible sleeve in a hand tool, secured at its opposed ends to slidably-engageable members of the hand tool. The loop portion is compressible radially inwardly by the application of slidably-engageable compressive force to the hand tool by the user, to uniformly and tightly crimp the stent onto the balloon catheter assembly. The loop portion further is releasable upon release by the user of the compressive force applied to the hand tool, to enable release of the stent crimped onto the balloon catheter assembly.

The crimping device enables the stent to be uniformly and tightly crimped onto the distal end of a balloon catheter, reducing the risk that the stent may slide off the catheter balloon portion. It further is easy to use in performing the stent crimping procedure.

In an exemplary method of crimping the stent onto the balloon portion of a catheter, the crimping device is designed to be hand-held and the crimping method performed by one person. The stent first is pre-loaded onto the balloon by sliding the stent over the deflated balloon. The stent and balloon catheter assembly are placed or positioned within the radially compressible device and supported therein so that the stent and balloon are positioned within the loop portion of the flexible sleeve. While the user holds the stent and balloon catheter assembly in one hand, a compressive force is applied using the other hand by applying slidingly engageable force with the crimping device. As the loop portion constricts in diameter, it will uniformly and tightly compress the stent onto the balloon portion of the catheter. Thereafter, the user releases the compressive force thereby releasing tension on the loop so that the stent, now tightly compressed onto the balloon portion of the catheter, can be removed from the crimping device.

These and other advantages ofthe invention will become more apparent from the following detailed description thereof when taken in conjunction with the accompanying drawings.

### BRIEF DESCRIPTION OF THE DRAWINGS

FIGURE 1 is a perspective view of an exemplary embodiment of the present invention, in which the slidably-engageable member is slidably moved into engagement with the receiving member;
FIG. 2 is a side elevational view of the exemplary embodiment of the present invention in the expanded loop portion condition.
FIG. 3 is a side elevational view of the exemplary embodiment of the present invention in which the loop portion of the hand tool is in compressed condition for crimping the stent onto the catheter balloon portion.
FIG. 4 is a side elevational view of the slidably engaging member and first securing member.
FIG. 5 is a top plan view of the slidably engaging member and first securing member, taken along line 5-5 of FIG. 4.
FIG. 6 is an end view of the first securing member taken along line 6-6 of FIG. 5.
FIG. 7 is a side elevational view of the receiving member and second securing member.
FIG. 8 is a top plan view of the receiving member and second securing member, taken along line 8-8 of FIG. 7.
FIG. 9 is an end view of the second securing member taken along line 9-9 of FIG. 8.
FIG. 10 is a perspective view depicting one embodiment of the loop portion.
FIG. 11 is a perspective view depicting an alternative embodiment of the loop portion.

### DETAILED DESCRIPTION OF THE PREFERRED EMBODIMENTS

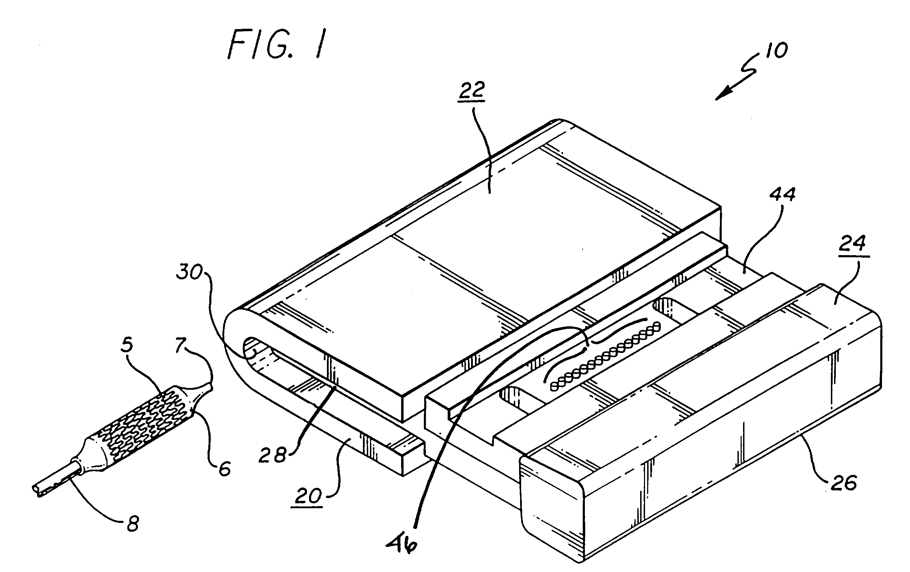

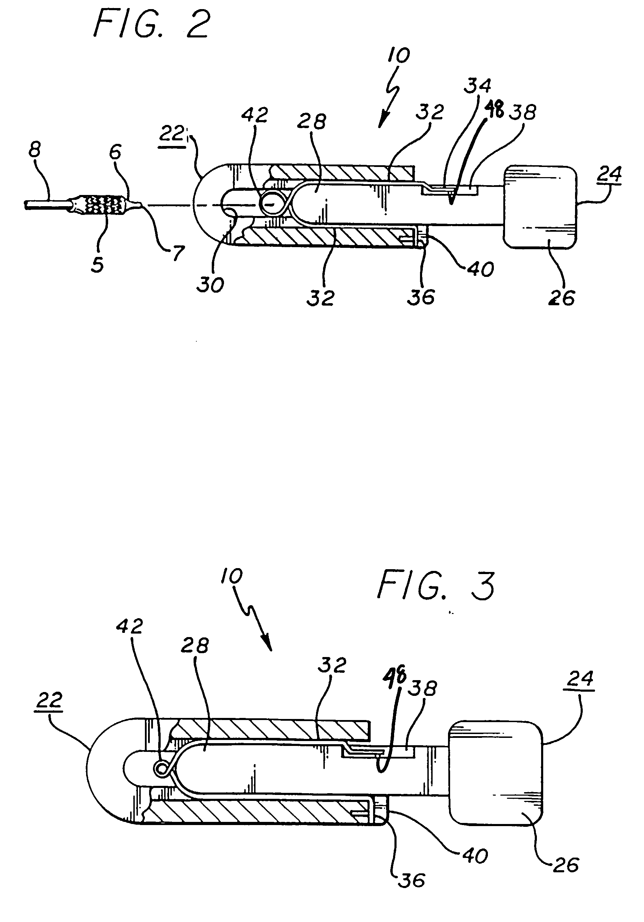

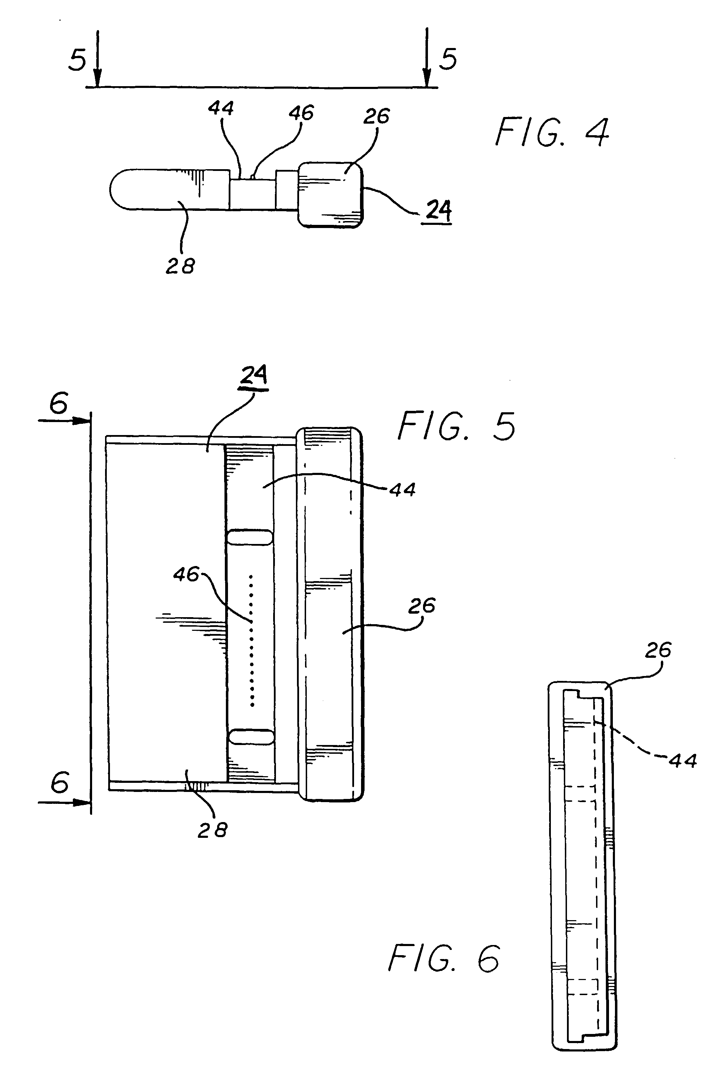

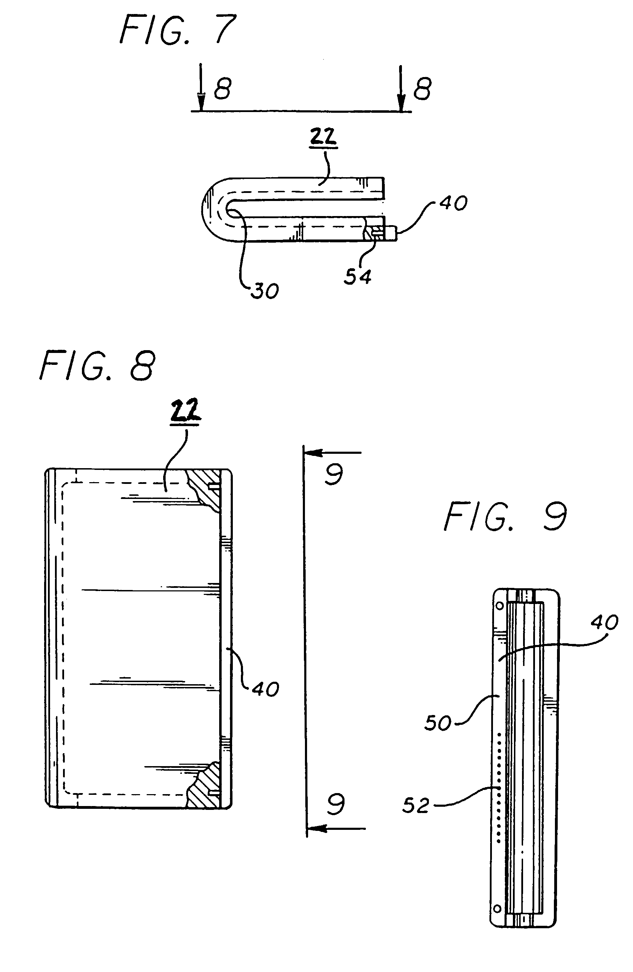

A device 10 comprises a tool 20 for enabling effective crimping of an intravascular stent 5 onto the collapsed balloon portion 6 adjacent the distal end 7 of a balloon catheter assembly 8. In the exemplary embodiment of the device 10, as shown in FIGS. 1-9, the tool 20 is adapted to be held in the hand of the user, so as to enable the stent 5 and the catheter 8 to be supported in the tool 20, and to enable the user to apply compressive force to the tool 20 to crimp the stent onto the catheter.

The tool 20 includes a receiving member 22, and a slidably-engageable member 24 that is slidably movable into engagement with the receiving member 22. The slidably-engaging member 24 includes a handle portion 26, and a projecting portion 28 slidably engageable with the receiving member. The receiving member 22 has a groove 30 therein. The projecting portion 28 of the slidably-engageable member 24 and the groove 30 of the receiving member 22 are engageable and generally complementary in shape. The receiving member 22 and the slidably engaging member 24 both preferably are translucent.

The tool 20 further includes a crimping member 32, secured at its ends to the slidably-engageable member 24 and the receiving member 22, for supporting the stent 5 and the catheter 8 therein. The crimping member 32 includes a first end 34, adapted to be secured to the slidably-engageable member 24, and a second end 36, at the end of the crimping member 32 opposite the first end, adapted to be secured to the receiving member 22. A first securing member 38 is adapted to secure the first end 34 to the slidably-engageable member 24, and a second securing member 40 is adapted to secure the second end 36 to the receiving member 22. The crimping member 32 further includes at least one compressible loop portion 42 wherein the portion of the balloon catheter assembly 8 with the stent 5 loaded thereon may be supported. The crimping member 32 is comprised ofcompressible material, such that upon sliding the slidably-engageable member 24 into engagement with the receiving member 22, the loop portion 42 is compressed radially inwardly to crimp the stent 5 onto the balloon portion 6. In other words, the diameter of the loop portion 42 decreases as the receiving member 22 and the slidably-engageable member 24 are squeezed together, thereby crimping the stent 5 onto the balloon portion 6. Upon release of the force being applied by the slidably-engageable member 24, by pulling the slidably-engageable member 24 out of engagement with the receiving member 22, the crimped stent 5 and the catheter may be removed from the loop portion 42. The compressible material of which the crimping member 32 is comprised preferably is a polyester film, such that sold under the trade name MYLAR by the E.I. duPont deNemours Company of Wilmington, Delaware. Ifthe receiving member 22 and the slidably-engageable member 24 are squeezed together repeatedly, the stent will be crimped ever tighter onto the balloon.

In the embodiment shown in FIGS. 1-9, the slidably-engageable member 24 includes a recessed portion 44 including a plurality of pegs 46 projecting therefrom, and the first securing member 38 includes a slot 48 for engaging the crimping member 32 and the plurality of pegs 46, to align and secure the crimping member 32 to the slidably-engageable member 24. The second securing member 40 includes a facing surface 50 including a plurality of pegs 52 projecting therefrom, and the receiving member 22 includes a slot 54 for engaging the crimping member 32 and the second securing member pegs 52, to align and secure the crimping member 32 to the receiving member 22.

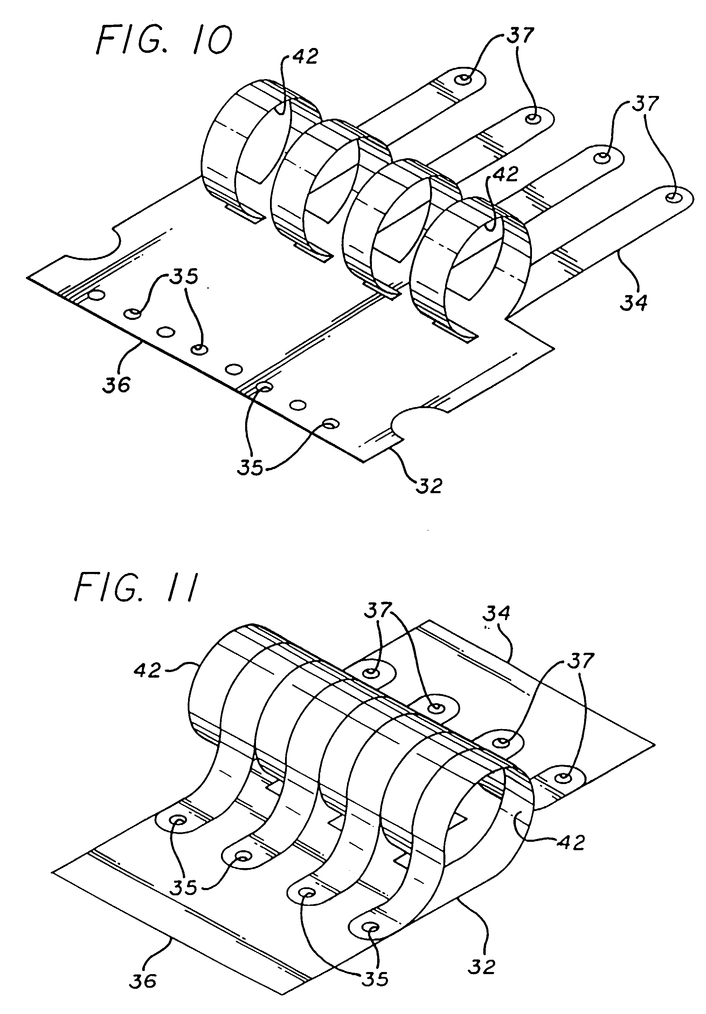

As seen in FIGS. 11 and 12, two preferred alternative embodiments of the crimping member 32 are depicted. The loop portion 42 includes a plurality of loops and it is sized to accept the stent 5 and the balloon portion 6 of the catheter prior to crimping. As the the slidably-engageable member 24 and the receiving member 22 are pushed together, the first end 34 of the crimping member and the second end 36 of the crimping member move in opposite directions, thereby constricting the loop portion 42 onto the stent and crimping it onto the balloon with increasing force. As is shown in FIGS. 10 and 11, in order to secure the crimping member 32 and to assist in placing the first end 34 and the second end 36 of the crimping member in tension, the holes 35 in the second end align with the pegs 52 in the second end and the holes 37 in the first end align with the pegs 46 in the first end. Thus, the first and second ends 34,36 ofthe crimping member are attached securely to the respective sets of pegs so that as the receiving member 22 and the slidably-engageable member 24 are squeezed together, the first and second ends 35,37 move with the pegs 46,52.

In operation, to load the stent 5 onto the collapsed balloon portion 6 of the balloon catheter assembly 8, the stent 5 is mounted over the balloon so that the stent overlies the balloon portion but is not crimped thereon. To enable the stent 5 to be crimped onto the catheter balloon portion 6, the stent and the catheter balloon portion may be inserted into and supported in the loop portion 42 of the tool-supporting crimping member 32. At this point, the stent 5 is not crimped onto the balloon because it has not been compressed.

To crimp the stent 5 onto the catheter balloon portion 6, the user ofthe tool 20 secures the ends 34,36 of the crimping member 32 to the slidably-engageable member 24 and the receiving member 22. The crimping member 32 is secured to the slidably-engageable member 24 by positioning the first end 34 of the crimping member 32 between the pegs 46 in the recessed portion 44 of the slidably-engageable member 24 and pressing the slot 48 in the first securing member 38 into engagement with the crimping member 32 and the pegs 46 in the slidably-engageable member 24. The crimping member 32 is secured to the receiving member 22 by positioning the second end 36 of the crimping member 32 between the pegs 52 in the facing surface 50 of the second securing member 40 and pressing the pegs 52 in the second securing member 40 into engagement with the crimping member 32 and the slot 54 in the receiving member 22.

The user of the tool 20 then may apply force to slide the slidably-engageable member 24 into engagement with the receiving member 22, such that as the projecting portion 28 of the slidably-engageable member 24 pushes the crimping member 32, secured at both of the ends 35,37, into the groove 30 of the receiving member 22. This motion then will move the first end 34 and the second end 36 in opposite directions, which causes the diameter of the loop portion 42 to become smaller and to compress radially inwardly, thereby compressing the stent 5 radially inwardly onto the catheter balloon portion 6.

After the stent 5 has been crimped onto the catheter balloon portion 6, the user may release the force applied to the crimping member 32 by pulling the slidably-engageable member 24 out of engagement with the receiving member 22. This motion allows the loop portion 42 to increase in diameter. The user then may release the crimping member 32 from being secured by the first securing member 38 and the second securing member 40, by disengaging the first member 38 from the crimping member 32 and the slidably-engageable member 24, and disengaging the second securing member 40 from the crimping member 32 and the receiving member 22, enabling the removal of the crimped stent and the catheter balloon portion from the loop portion 42. The balloon catheter assembly 8, with the stent 5 crimped thereon, then is ready for insertion into the body ofthe patient for deployment of the stent therein.

A novel feature of the present invention is the ability of the crimping tool to vary the constriction of various parts of the stent. Thus, the stent will be crimped more tightly onto the balloon in some places, localizing the traction (interface) between the stent and the balloon. Even though there are variations in the crimping force experienced by the stent, the force remains within the bounds of uniformity. In the case of a coronary artery stent, the crimped stent may have diameters along its length in the range of 0.0762 mm to 0.127 mm (0.003 inch to 0.005 inch) and still be considered a uniform crimp with good traction or good holding force on the balloon.

While in the preferred embodiment the stent described is intended to be an intraluminal vascular prosthesis for use within a blood vessel, and the balloon delivery catheter is ofthe same or similar to that used in therapeutic coronary angioplasty, it will be appreciated by those skilled in the art that modifications may be made to the present invention to allow the present invention to be used to crimp any type of prosthesis. The present invention is not limited to stents that are deployed in a patient's vasculature, but has wide applications to crimping any type of graft, prosthesis, liner or similar structure. Further, the stent may be delivered not only into coronary arteries, but into any body lumen. Other modifications can be made to the present invention by those skilled in the art without departing from the scope thereof.

## Claims

1. A hand-held device (10) for crimping a stent (5) onto a balloon catheter assembly (8), comprising:
means for supporting (20) a portion of the balloon catheter assembly on which the stent may be loaded, the supporting means (20) having a compressible portion (32) which is compressible radially inwardly upon the application of force thereto to crimp the stent (5) onto the catheter portion (8), and is releasable upon release of the applied force to release the crimped stent and catheter portion; and
means for enabling force to be applied (22,24) to the supporting means (20) and to be slidably movable and engageable, so as to enable the user to apply slidably-engageable compressive force thereto to generate compression, radially inwardly, of the compressible portion (32) of the supporting means (20), for crimping the stent (5) onto the catheter portion (8).

2. A device as in claim 1, wherein the compressible portion (32) ofthe supporting means (20) comprises a sleeve including at least one loop (42) for supporting the stent (5) and the catheter portion (8) and for enabling crimping ofthe stent (5) onto the catheter portion (8).

3. A device as in claim 1, wherein the force-application-enabling means (22,24) comprises means for receiving (22) the compressible portion (32) of the supporting means (20).

4. A device as in claim 2, wherein the sleeve is comprised of a flexible material.

5. A device as in claim 3, wherein the force-application-enabling means (22,24) further comprises means capable of slidably engaging (24) the receiving means (22) so as to compress the compressible portion (32) of the supporting means (20).

6. A device as in claim 3, wherein the receiving means (22) includes a groove (30) therein.

7. A device as in claim 3, wherein the receiving means (22) is comprised of a translucent material.

8. A device as in claim 4, wherein the flexible sleeve material comprises a polyester film.

9. A device as in claim 5, wherein the force-application-enabling means (22,24) further comprises means for securing (38,40) the compressible portion (32) of the supporting means (20) to the slidably-engageable means (24) and the receiving means (22).

10. A device as in claim 5, wherein the slidably-engagable means (24) includes a handle portion (26), and a projecting portion (28) slidably engageable with the receiving means (22).

11. A device as in claim 9, wherein the compressible portion (32) ofthe supporting means (20) includes a first portion (34), and the securing means (38,40) comprises means for securing (40) the second portion (36) ofthe compressible portion (32) ofthe supporting means (20) to the receiving means (22).

12. A device as in claim 10, wherein the receiving means (22) includes a groove (30) therein, and the slidably-engagable means projecting portion (28) and the receiving means groove (30) are generally complementary in shape.

13. A device as in claim 11, wherein the compressible portion (32) of the supporting means (20) further comprises a first portion (34), and the securing means (38,40) further comprises means for securing (38) the first portion (34) of the compressible portion (32) of the supporting means (20) to the slidably-engageable means (24).

14. A method of crimping an intravascular stent (5) onto a balloon catheter assembly (8), comprising:
placing a portion of the balloon catheter assembly (8), on which the stent is positioned, in a radially compressible device (10);
applying slidably-engageable compressive force to compress the compressible device (10) radially inwardly, to crimp the stent (5) onto the balloon catheter assembly (8); and
releasing the compressive force, to enable removal ofthe crimped stent (5) and the balloon catheter assembly (8).

15. A method as in claim 14, wherein the step of placing the stent (5) and balloon catheter assembly (8) in the radially compressible device (10) comprises placing the balloon catheter assembly (8) in a compressible portion (32) ofmeans for supporting (20) the stent and the balloon catheter assembly, in a device adapted to be held in the hand of the user, so as to enable the user to apply the compressive force thereto.

16. A method as in claim 14, wherein the step of supporting the stent (5) and the balloon catheter assembly (8) in the radially compressible device (10) comprises supporting the stent and the balloon catheter assembly in at least one loop (42) in a sleeve, and the step of applying force to the compressible device comprises applying force to the at least one loop (42) in the sleeve.

17. A method as in claim 14, wherein the step of applying compressive force to the compressible device comprises applying force to a slidably-engageable member (24) and a receiving member (22) to which the compressible portion (32) stent (5) and the balloon catheter assembly supporting means (20) is secured, and compressing the slidably-engageable member (24) and the receiving member (22) so as to compress the supporting means (20), to crimp the stent (5) onto the catheter (8).

18. An intravascular stent crimped onto a balloon catheter assembly portion, comprising:
a balloon catheter assembly portion (8); and
a stent (5) crimped onto the balloon catheter assembly portion (8) by compressive force applied thereto through a device (10) adapted to be held in the hand of the user comprising a slidably-engageable member (24) and a receiving member (22).

19. A crimping tool (20) for uniformly and tightly crimping a stent (5) onto the balloon portion of a catheter (8), comprising:
a crimping member (32) having a flexible sleeve, the flexible sleeve having a loop portion (42) capable of changing from a first larger diameter to a second smaller diameter;
a receiving member (22) configured for slidable engagement with a projecting member (28), the receiving member (22) and the projecting member (28) having generally complimentary shapes ergonomically designed to fit the hand of a user;
the flexible sleeve having a first end (34) and a second end (36), the ends (34, 36) being attached to the projecting member (28) so that when the stent (5) and balloon portion (8) are positioned within the loop portion (42), the user will slidingly engage the receiving member (22) and the projecting member (28) to pull on the ends (34,36) of the flexible sleeve which in turn will constrict the loop portion (42) from the first larger diameter to the second smaller diameter, thereby applying compressive forces to tightly crimp the stent (5) onto the balloon portion of the catheter (8).

20. A crimping tool (20) as in claim 19, wherein the receiving member (22) has a groove (30) configured for receiving the complimentarily-shaped projecting member (28).

21. A crimping tool (20) as in claim 19, wherein the crimping tool (20) is formed from a substantially rigid material.

22. A crimping tool (20) as in claim 19, wherein the flexible sleeve ofthe crimping member (32) is formed a flexible plastic.

23. A crimping tool (20) as in claim 19, wherein the first end (34) of the flexible sleeve of the crimping member (32) is attached to the receiving member (22) by a second securing member (40) and the second end (36) of the flexible sleeve is attached to the projecting member (28) by a first securing member (38).

24. A crimping tool as in claim 23, wherein the first end (34) and the second end (36) ofthe flexible sleeve are aligned by a plurality ofpegs (46,52) extending through the ends (34,36) and into the second and first securing members (40,38).

25. A crimping tool (20) as in claim 19, wherein the flexible sleeve ofthe crimping member (32) has a plurality of loop portions (42) capable of changing from the first larger diameter to the second smaller diameter in order to apply a crimping force to crimp the stent (5) onto the balloon portion of the catheter (8).

26. A crimping tool (20) as in claim 25, wherein the crimping force applied to the stent (5) by the plurality of loop portions (42) varies along the length of the stent (5).
